(19) Europäisches Patentamt
European Patent Office
Office européen des brevets

(11) EP 4 529 830 A1

(12) EUROPEAN PATENT APPLICATION

(43) Date of publication:
02.04.2025 Bulletin 2025/14

(51) International Patent Classification (IPC):
A61B 5/00 (2006.01)    A61B 5/145 (2006.01)
A61B 5/1495 (2006.01)

(21) Application number: 23214169.7

(22) Date of filing: 05.12.2023

(52) Cooperative Patent Classification (CPC):
A61B 5/1495; A61B 5/0075; A61B 5/14542;
A61B 5/443; A61B 2560/0238

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.09.2023 US 202363540960 P

(71) Applicant: Koninklijke Philips N.V.
5656 AG Eindhoven (NL)

(72) Inventors:
• KOSTAKIS, Dimitri George
Eindhoven (NL)
• BRADY, Leigh
Eindhoven (NL)

(74) Representative: Philips Intellectual Property &
Standards
High Tech Campus 52
5656 AG Eindhoven (NL)

(54) **SYSTEM AND METHODS FOR DYNAMIC CALIBRATION OF PULSE OXIMETRY DEVICES BASED ON SKIN PIGMENTATION**

(57) Provided herein are systems and methods of dynamically calibrating the measurement performance of an individual pulse oximetry device based on the skin pigmentation of the patient at the application site. Unlike conventional approaches, the pulse oximetry devices of the present disclosure may be calibrated any number of times at the time of use such that the calibration is specific to the particular individual being monitored. The systems and methods described herein utilize (i) a pulse oximetry device having a first light source, a spectroscopy device used to measure light from the first light source, a set of second light sources, and a light-sensitive element used to measure light from the set of second light sources, and (ii) a calibration module that uses the measurements from the spectroscopy device and the light-sensitive element to select a predefined R-curve for an individual and take a pulse oximetry measurement.

FIG. 3

- 310 → SUBJECT TARGET TO LIGHT USING A FIRST LIGHT SOURCE
- 320 → MEASURE LIGHT TRANSMITTED / REFLECTED BY THE TARGET USING A SPECTROSCOPY DEVICE
- 330 → SUBJECT TARGET TO AT LEAST TWO DIFFERENT WAVELENGTHS OF LIGHT USING A SET OF LIGHT SOURCES
- 340 → MEASURE LIGHT TRANSMITTED / REFLECTED BY THE TARGET USING A LIGHT-SENSITIVE ELEMENT
- 350 → DETERMINE A BLOOD OXYGEN SATURATION MEASUREMENT BASED ON THE LIGHT MEASURED BY THE SPECTROSCOPY DEVICE AND THE LIGHT-SENSITIVE ELEMENT
- 360 → DISPLAY MEASUREMENT FROM PULSE OXIMETRY DEVICE

EP 4 529 830 A1

## Description

FIELD OF THE INVENTION

The present disclosure relates generally to pulse oximetry devices and methods of performing pulse oximetry, and more specifically to systems and methods that dynamically calibrate a pulse oximetry device based on skin pigmentation.

BACKGROUND OF THE INVENTION

Pulse oximetry is a frequently-used patient measurement technique that measures blood oxygen saturation ($SpO_2$), and generally involves sending light through the tissues of a patient, such as through the patient's finger, toe, ear, nose, and/or other extremity, and measuring the resulting light passing through those tissues. The result is an oxygen level typically displayed as a percentage on a patient monitoring system. Typically, the $SpO_2$ measurement performance of a pulse oximetry device must be tested and verified against industry standards. In particular, a typical pulse oximetry device must perform well for a potentially diverse group of patients with different skin types, conditions, and pigmentation. Thus, in conventional pulse oximetry applications, the $SpO_2$ measurement performance of a specific pulse oximetry device may be calibrated by performing a study that includes a cross-section of individuals.

SUMMARY OF THE INVENTION

While pulse oximetry is a frequently-used patient monitoring technique to measure blood oxygen saturation, conventional pulse oximetry devices are calibrated in a static manner, i.e., a calibration study is performed for a particular model of pulse oximetry devices before its release onto the market and the calibration results are embedded in the device for future use. In this way, even though the pulse oximetry device may be used on different patients, the calibration of the device remains the same. Provided herein are systems and methods of dynamically calibrating the $SpO_2$ measurement performance of an individual pulse oximetry device based on the skin pigmentation of the patient and the application site.

According to an embodiment of the present disclosure, a system for dynamically calibrating pulse oximetry measurements based on skin pigmentation is provided. The system can include a pulse oximetry device comprising: a first light source configured to subject a target region of an individual to a predetermined amount of light; a spectroscopy device configured to measure light produced by the first light source, wherein the light measured by the spectroscopy device includes at least a portion of the predetermined amount of light that is either transmitted through and/or reflected by the target region of the individual; a set of second light sources configured to subject the target region of the individual to at least two different wavelengths of light; and a light-sensitive element configured to measure light produced by the set of second light sources, wherein the light measured by the light-sensitive element includes light that is either transmitted through and/or reflected by the target region of the individual. The system can further include a calibration module in communication with the pulse oximetry device. The calibration module can be configured to receive electrical signals generated by the spectroscopy device and the light-sensitive element based on the light measured respectively, and can be further configured to determine a blood oxygen saturation measurement based on the electrical signals received from the spectroscopy device and the light-sensitive element.

In an aspect, the electrical signals generated by the spectroscopy device and the light-sensitive element can be representative of an amount of light measured by the spectroscopy device and the light-sensitive element, respectively.

In an aspect, the pulse oximetry device can be calibrated based on the light measured by the spectroscopy device and the blood oxygen saturation measurement can be determined based on the light measured by the light-sensitive element.

In an aspect, the blood oxygen saturation measurement may be determined based on the light measured by the light-sensitive element and an R-curve selected based on the light measured by the spectroscopy device.

In an aspect, the calibration module can include an R-curve database storing a plurality of R-curves, wherein each R-curve corresponds to one or a range of skin pigmentations.

In an aspect, the calibration module can be configured to select an R-curve from the plurality of R-curves stored in the R-curve database based on the light measured by the spectroscopy device.

In an aspect, the calibration module can be configured to determine the blood oxygen saturation measurement based on the electrical signals received from the light-sensitive element when the target region was not subjected to the predetermined amount of light produced by the first light source.

According to another embodiment of the present disclosure, a computer program product is provided. The computer program product can include a non-transitory computer-readable storage medium having stored thereon computer-readable instructions that, when executed by one or more processors, cause the one or more processors to perform the following operations: (i) subjecting a target region of an individual to a predetermined amount of light using a first light source of a pulse oximetry device; (ii) measuring light produced by the first light source using a spectroscopy device, wherein the light measured by the spectroscopy device includes at least a portion of the predetermined amount of light that is either transmitted through and/or reflected by the target region of the individual; (iii) subjecting the target region of the individual to at least two different wavelengths of light using a set of second light sources of the pulse oximetry device; (iv) measuring light produced by the set of second light sources using a light-sensitive element, wherein the light measured by the light-sensitive element includes light that is either transmitted through and/or reflected by the target region of the individual; and (v) determining a blood oxygen saturation measurement for the individual based on the light measured by the spectroscopy device and the light measured by the light-sensitive element.

In an aspect, determining the blood oxygen saturation measurement for the individual can include: (a) determining a skin pigmentation value for the target region of the individual based on the light measured by the spectroscopy device when the target region was subjected to the predetermined amount of light produced by the first light source; (b) selecting an R-curve based on the skin pigmentation value determined for the target region of the individual; and (c) determining the blood oxygen saturation measurement for the individual based on the R-curve selected and the light measured by the light-sensitive element when the target region was subjected to the at least two different wavelengths of light produced by the set of second light sources.

In an aspect, the R-curve can be selected from an R-curve database storing a plurality of predefined R-curves, wherein each R-curve corresponds to one or a range of skin pigmentation values.

In an aspect, the blood oxygen saturation measurement can be determined based on the light measured by the light-sensitive element when the target region was not subjected to the predetermined amount of light produced by the first light source.

According to still another embodiment of the present disclosure, a method for measuring a physiological parameter using a pulse oximetry device comprising a first light source, a spectroscopy device, a set of second light sources, and a light-sensitive element. The method can include: subjecting a target region of an individual to a predetermined amount of light using the first light source of the pulse oximetry device; measuring light produced by the first light source using the spectroscopy device of the pulse oximetry device, wherein the light measured by the spectroscopy device includes at least a portion of the predetermined amount of light that is either transmitted through and/or reflected by the target region of the individual; subjecting the target region of the individual to at least two different wavelengths of light using the set of second light sources of the pulse oximetry device; measuring light produced by the set of second light sources using the light-sensitive element of the pulse oximetry device, wherein the light measured by the light-sensitive element includes light that is either transmitted through and/or reflected by the target region of the individual; and determining a blood oxygen saturation measurement for the individual based on the light measured by the spectroscopy device and the light measured by the light-sensitive element.

In an aspect, determining the blood oxygen saturation measurement for the individual can include: determining a skin pigmentation value for the target region of the individual based on the light measured by the spectroscopy device when the target region was subjected to the predetermined amount of light produced by the first light source; selecting an R-curve based on the skin pigmentation value determined for the target region of the individual; and determining the blood oxygen saturation measurement for the individual based on the R-curve selected and the light measured by the light-sensitive element when the target region was subjected to the at least two different wavelengths of light produced by the set of second light sources.

In an aspect, the R-curve can be selected from an R-curve database storing a plurality of predefined R-curves, wherein each R-curve corresponds to one or a range of skin pigmentation values.

In an aspect, the blood oxygen saturation measurement can be determined based on the light measured by the light-sensitive element when the target region was not subjected to the predetermined amount of light produced by the first light source.

These and other aspects of the various embodiments will be apparent from and elucidated with reference to the embodiments described hereinafter.

BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.

Fig. 1 is a block diagram illustrating a system for dynamically calibrating the $SpO_2$ measurement performance of a pulse oximetry device in accordance with aspects of the present disclosure.

Fig. 2 is a block diagram illustrating a calibration module configured to dynamically calibrate the $SpO_2$ measurement performance of a pulse oximetry device in accordance with aspects of the present disclosure.

Fig. 3 is a flowchart illustrating a method of dynamically calibrating the SpO2 measurement performance of a pulse oximetry device in accordance with aspects of the present disclosure.

DETAILED DESCRIPTION OF EMBODIMENTS

As mentioned above, it is recognized and appreciated by the present disclosure that blood oxygen saturation ($SpO_2$) measurement performance can be affected by a variety of factors, including the skin pigmentation of the target site where the pulse oximetry device is coupled with the patient. Conventional ap-

proaches calibrate $SpO_2$ measurement performance in a static manner by performing a desaturation study that includes a number of individuals with different skin pigmentations. However, these approaches are static in nature and do not account for the effects that the skin pigmentation of a specific individual will have on the accuracy of $SpO_2$ measurement.

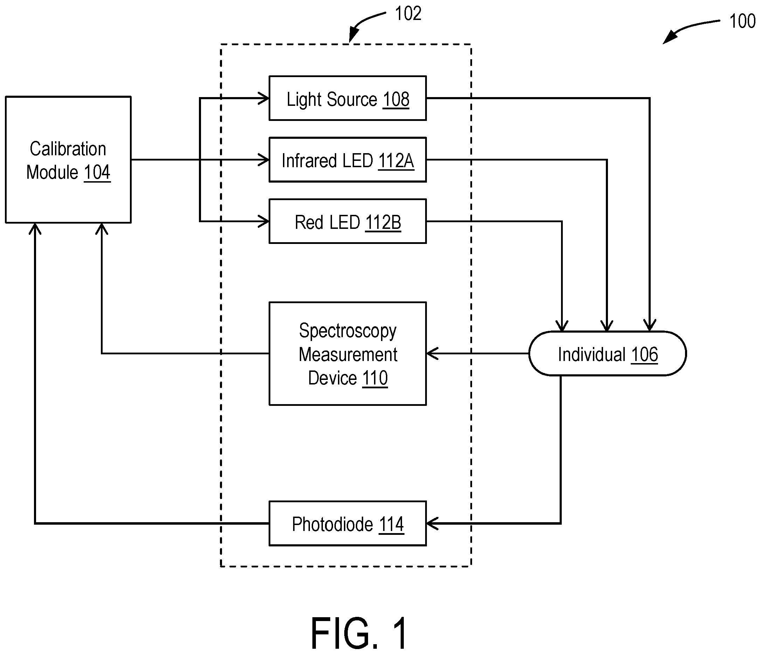

Accordingly, provided herein are systems and methods of measuring the blood oxygen saturation of an individual that dynamically calibrate based on the skin pigmentation of the individual at the target measurement site. With reference to Fig. 1, a system 100 for dynamically calibrating pulse oximetry measurements based on skin pigmentation is illustrated in accordance with various aspects of the present disclosure. As shown, the system 100 can include a pulse oximetry device 102 in communication with a calibration module 104. The pulse oximetry device 102 can be coupled with an individual 106, such as a patient for whom as pulse oximetry measurement may be taken. In particular embodiments, the pulse oximetry measurement is a blood oxygen saturation ($SpO_2$) measurement, and the pulse oximetry device 102 is coupled to a target site on the individual 106, such as the individual's finger, toe, ear, nose, and/or other extremity.

In embodiments, the pulse oximetry device 102 can include a first light source 108 configured to subject the target site / region of the individual 106 to a predetermined amount of light. The first light source 108 can be, for example and without limitation, a color light-emitting diode (LED) or other light source, and can be arranged such that the first light source 108 directs an amount of light (at a particular wavelength, intensity, and/or duration) towards the target region of the individual 106.

In embodiments, the pulse oximetry device 102 can also include a spectroscopy measurement device 110 configured to measure light produced by the first light source 108, including at least a portion of the predetermined amount of light that is either transmitted through and/or reflected by the target region of the individual 106. In particular embodiments, the spectroscopy measurement device 110 produces electrical signals based on the light received from the first light source 108, which are transmitted to and received by the calibration module 104. As discussed in more detail below, the calibration module 104 can be configured to determine a skin pigmentation value for the target region of the individual 106 based on the light measured by the spectroscopy device 110 (i.e., based on the electrical signals that are generated by the spectroscopy device 110 based on the light received from the first light source 108).

In embodiments, the pulse oximetry device 102 can also include a set of second light sources 112A, 112B configured to subject the target region of the individual 106 to at least two different wavelengths of light. For example, in particular embodiments, the set of second light sources 112A, 112B can include an infrared LED light source 112A configured to produce light in the infrared spectrum, and a red LED light source 112B configured to produce light in the red visible light spectrum. The set of second light sources 112A, 112B can be arranged such that light produced by the light sources 112A, 112B is directed towards the target region of the individual 106.

The pulse oximetry device 102 can also include a light-sensitive element 114 configured to measure light produced by the set of second light sources 112A, 112B, including light from the set of second light sources 112A, 112B that is either transmitted through and/or reflected by the target region of the individual 106. In embodiments, the light-sensitive element 114 can be, for example and without limitation, one or more photodiodes and/or photodetectors. In particular embodiments, the light-sensitive element 114 produces electrical signals based on the light received from the set of second light sources 112A, 112B, which are transmitted to and received by the calibration module 104. As discussed in more detail below, the calibration module 104 can be configured to determine a pulse oximetry measurement (e.g., a blood oxygen saturation measurement) for the individual 106 based on the light measured by the light-sensitive element 114 and the spectroscopy device 110 (i.e., based on the electrical signals that are generated by the light-sensitive element 114 and the spectroscopy device 110).

In certain embodiments, the one or more components of the pulse oximetry device 102 may be located in an accessory physically coupled to the individual 106. For example, one or more light sources 108, 112A, 112B, the spectroscopy device 110, and/or the photodiode 114 may be located within a housing that is clipped onto the individual 106. However, it should be appreciated that one or more of these components may also be located remotely from the accessory / housing attached to the individual 106. For example, in some embodiments, one or more of the light sources 108, 112A, 112B may be located remotely from the housing that is clipped onto the individual 106, and one or more optical cables (not shown) are used to direct the light from these light sources 108, 112A, 112B to the target region of the individual 106.

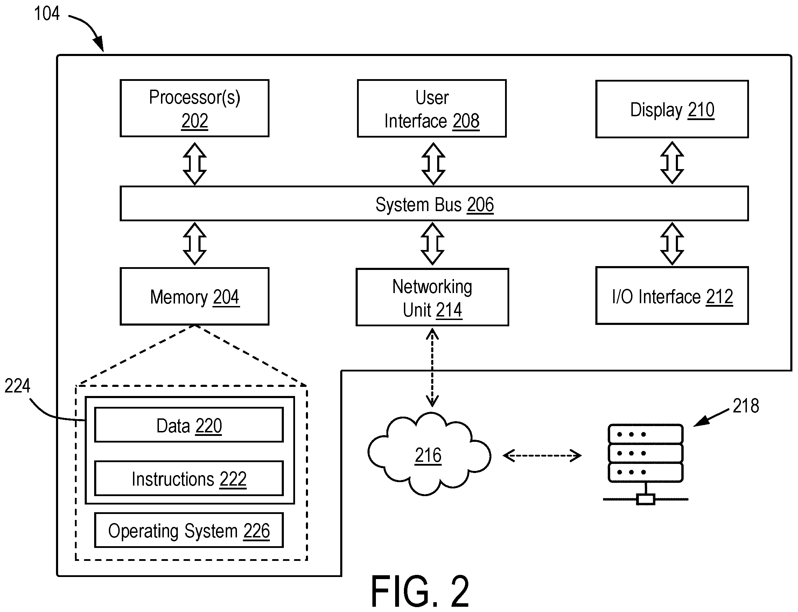

Turning to Fig. 2, a block diagram of a calibration module 104 that may form a part of a system 100 as discussed above is illustrated in accordance with various aspects of the present disclosure. The calibration module 104 can connected to and/or otherwise in communication with the pulse oximetry device 102. In particular embodiments, the calibration module 104 can be configured to dynamically calibrate the pulse oximetry measurements of the pulse oximetry device 104 based on skin pigmentation at the target site of the individual 106.

In the example of Fig. 2, the calibration module 104 can include one or more processors 202 and a computer-readable memory 204 interconnected and/or in communication via a system bus 206 containing conductive circuit pathways through which instructions (e.g.,

machine-readable signals) may travel to effectuate communication, tasks, storage, and the like. The calibration module 104 can be connected to a power source (not shown), which can include an internal power supply and/or an external power supply. In embodiments, the calibration module 104 can also include one or more additional components, such as a user interface 208, a display 210, an input/output (I/O) interface 212, a networking unit 214, and the like, including combinations thereof. As shown, each of these components may be interconnected and/or in communication via the system bus 206, for example.

In embodiments, the one or more processors 202 can include one or more highspeed data processors adequate to execute the program components described herein and/or perform one or more operations of the methods described herein. The one or more processors 202 may include a microprocessor, a multi-core processor, a multithreaded processor, an ultra-low voltage processor, an embedded processor, and/or the like, including combinations thereof. The one or more processors 202 can include multiple processor cores on a single die and/or may be a part of a system on a chip (SoC) in which the processor 202 and other components are formed into a single integrated circuit, or a single package. That is, the one or more processors 202 may be a single processor, multiple independent processors, or multiple processor cores on a single die.

In embodiments, the user interface 208 may be configured to receive various forms of input from a user associated with the calibration module 104. The user interface 208 can include, but is not limited to, one or more of a keyboard, keypad, trackpad, trackball(s), capacitive keyboard, controller (e.g., a gaming controller), computer mouse, computer stylus / pen, a voice input device, and/or the like, including combinations thereof.

In embodiments, the display device 210 may be configured to display information, including text, graphs, and/or the like. In particular embodiments, the display device 210 may be configured to display a skin pigmentation value and/or one or more pulse oximetry measurements, such as a blood oxygen saturation measurement. The display device 210 can include, but is not limited to, a liquid crystal display (LCD), a light-emitting diode (LED) display, a touch screen or other touch-enabled display, a foldable display, a projection display, and so on, or combinations thereof.

In embodiments, the input/output (I/O) interface 212 may be configured to connect and/or enable communication with one or more peripheral devices (not shown), including but not limited to additional machine-readable memory devices, diagnostic equipment, and other attachable devices (not shown). The I/O interface 212 may include one or more I/O ports that provide a physical connection to the one or more peripheral devices. In some embodiments, the I/O interface 212 may include one or more serial ports.

In embodiments, the networking unit 214 may include one or more types of networking interfaces that facilitate wired and/or wireless communication between the calibration module 104 and one or more external devices. That is, the networking unit 214 may operatively connect the calibration module 214 to one or more types of communications networks 216, which can include a direction interconnection, the Internet, a local area network ("LAN"), a metropolitan area network ("MAN"), a wide area network ("WAN"), a wired or Ethernet connection, a wireless connection, a cellular network, and similar types of communications networks, including combinations thereof. In some embodiments, the calibration module 104 may communicate with one or more remote / cloud-based servers and/or cloud-based services, such as remote server 218, via the communications network 216.

In embodiments, the memory 204 can be variously embodied in one or more forms of machine accessible and machine-readable memory. In some embodiments, the memory 204 includes a storage device (not shown), which can include, but is not limited to, a non-transitory storage medium, a magnetic disk storage, an optical disk storage, an array of storage devices, a solid-state memory device, and/or the like, as well as combinations thereof. The memory 204 may also include one or more other types of memory, such as dynamic random-access memory (DRAM), static random-access memory (SRAM), erasable programmable read-only memory (EPROM), electrically erasable programmable read-only memory (EEPROM), Flash memory, and/or the like, as well as combinations thereof. In embodiments, the memory 204 may include one or more types of transitory and/or non-transitory memory.

The calibration module 104 can be configured by software components stored in the memory 204 to perform one or more processes of the methods described herein. More specifically, the memory 204 can be configured to store data / information 220 and computer-readable instructions 222 that, when executed by the one or more processors 202, causes the calibration module 104 to dynamically calibrate and perform a pulse oximetry measurement of an individual 106. Such data 220 and the computer-readable instructions 222 stored in the memory 204 may form a calibration module package 224 that may be incorporated into, loaded from, loaded onto, or otherwise operatively available to and from the calibration module 104. Thus, in some embodiments, the calibration module package 224 and/or one or more individual software packages may be stored in a local storage device of the memory 204. However, in other embodiments, the calibration module package 224 and/or one or more individual software packages may be loaded onto and/or updated from a remote server or service, such as server 218, via the communications network 216.

The calibration module 104 may also include an operating system component 226, which may be stored in the memory 204. The operating system component 224 may be an executable program facilitating the operation of the calibration module 104. Typically, the operating system component 226 can facilitate access of the I/O interface 212, network interface 214, the user interface 208, and the display 210, and can communicate or control other components of the calibration module 104.

Accordingly, provided herein is a computer program product 224 comprising a non-transitory computer-readable storage medium 204 having stored thereon computer-readable instructions 222 that, when executed by one or more processors (such as processors 202), cause the one or more processors to perform one or more operations of the methods described below. For example, in specific embodiments, the computer-readable storage medium 204 may include computer-readable instructions 222 that, when executed by one or more processors (such as processors 202), cause the one or more processors to perform the following operations: (i) subjecting a target region of an individual to a predetermined amount of light using a first light source of a pulse oximetry device; (ii) measuring light produced by the first light source using a spectroscopy device, wherein the light measured by the spectroscopy device includes at least a portion of the predetermined amount of light that is either transmitted through and/or reflected by the target region of the individual; (iii) subjecting the target region of the individual to at least two different wavelengths of light using a set of second light sources of the pulse oximetry device; (iv) measuring light produced by the set of second light sources using a photodiode, wherein the light measured by the photodiode includes light that is either transmitted through and/or reflected by the target region of the individual; and (v) determining a blood oxygen saturation measurement for the individual based on the light measured by the spectroscopy device and the light measured by the photodiode.

More particularly, the computer-readable storage medium 204 can include computer-readable instructions 222 that, when executed by one or more processors (such as processors 202), cause the one or more processors to perform a method for measuring a physiological parameter using a pulse oximetry device 102 in accordance with the various aspects described herein.

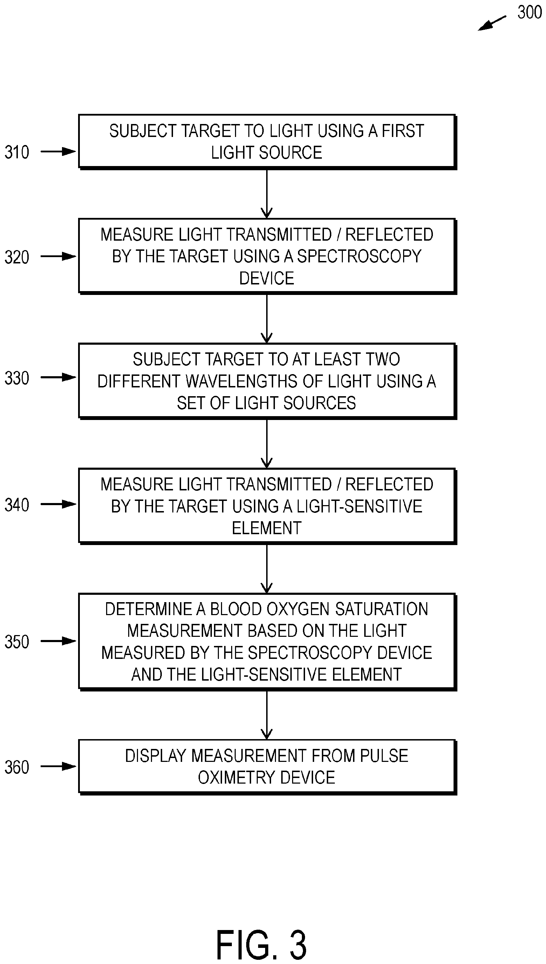

For example, with reference to Fig. 3, a method 300 for measuring a physiological parameter using a pulse oximetry device 102 comprising a first light source 108, a spectroscopy device 110, a set of second light sources 112A, 112B, and a light-sensitive element 114 is illustrated in accordance with certain aspects of the present disclosure. As shown, the method 300 can include: in a step 310, subjecting a target region of an individual to a predetermined amount of light using the first light source of the pulse oximetry device; in a step 320, measuring light produced by the first light source using the spectroscopy device of the pulse oximetry device, wherein the light measured by the spectroscopy device includes at least a portion of the predetermined amount of light that is either transmitted through and/or reflected by the target region of the individual; in a step 330, subjecting the target region of the individual to at least two different wavelengths of light using the set of second light sources of the pulse oximetry device; in a step 340, measuring light produced by the set of second light sources using the photodiode of the pulse oximetry device, wherein the light measured by the photodiode includes light that is either transmitted through and/or reflected by the target region of the individual; and in a step 350, determining a blood oxygen saturation measurement for the individual based on the light measured by the spectroscopy device and the light measured by the photodiode. In embodiments, the method 300 can further include, in a step 360, displaying on a display the measurement from the pulse oximetry device (e.g., the blood oxygen saturation measurement).

In embodiments, step 310 of the method 300 includes subjecting a target region of an individual to light using a first light source 108 of a pulse oximetry device 102. The target region (i.e., target site) can be, but is not limited to, the individual's finger, toe, ear, nose, and/or other extremity. In some embodiments, the first light source 108 can be, for example and without limitation, a color light-emitting diode (LED) or other light source, and can be arranged such that the first light source 108 directs an amount of light (at a particular wavelength, intensity, and/or duration) towards the target region of the individual 106. In specific embodiments, the pulse oximetry device 102 may be coupled to the individual 106 such that the first light source 108 can direct light towards the target region.

In embodiments, step 320 of the method 300 includes measuring the light produced by the first light source 108 after being transmitted through and/or reflected by the individual 106 at the target region using a spectroscopy measurement device 110. Put another way, the spectroscopy measurement device 110 can be configured to measure light produced by the first light source 108, including at least a portion of the predetermined amount of light that is either transmitted through and/or reflected by the target region of the individual 106. In particular embodiments, the spectroscopy measurement device 110 produces electrical signals based on the light received from the first light source 108, which may be transmitted to and received by another device, such as a patient monitoring comprising a calibration module 104.

In embodiments, step 330 of the method 300 includes subjecting the target region of the individual 106 to at least two different wavelengths of light using a set of second light sources 112A, 112B of the pulse oximetry device 102. In certain embodiments, the set of second light sources 112A, 112B can include an infrared LED light source 112A configured to produce light in the infrared spectrum, and a red LED light source 112B configured to produce light in the red visible light spectrum. The set of second light sources 112A, 112B can be arranged such that light produced by the light sources 112A, 112B is directed towards the target region of the individual 106. That is, the pulse oximetry device 102 may be coupled to the individual 106 such that the set of second light sources 112A, 112B can direct light towards the target region.

In embodiments, step 340 of the method 300 includes measuring the light produced by the set of second light sources 112A, 112B after being transmitted through and/or reflected by the individual 106 at the target region using a light-sensitive element 114. Put another way, the light-sensitive element 114 can be configured to measure light produced by the set of second light sources 112A, 112B that was either transmitted through and/or reflected by the target region of the individual 106. In embodiments, the light-sensitive element 114 can be, for example and without limitation, one or more photodiodes and/or photodetectors. In particular embodiments, the light-sensitive element 114 produces electrical signals based on the light received from the set of second light sources 112A, 112B, which may be transmitted to and received by another device, such as a patient monitoring comprising a calibration module 104.

In embodiments, step 350 of the method 300 includes determining a pulse oximetry measurement based on the light measured by the spectroscopy device 110 and the light-sensitive element 114. In particular embodiments, step 350 can include determining a pulse oximetry measurement (e.g., a blood oxygen saturation measurement) for the individual 106 based on the light measured by the light-sensitive element 114 and the spectroscopy device 110 (i.e., based on the electrical signals that are generated by the light-sensitive element 114 and the spectroscopy device 110). In certain embodiments, the pulse oximetry device 102 may be calibrated based on the light produced by the first light source 108 and measured by the spectroscopy device 110, and the pulse oximetry measurement (e.g., the blood oxygen saturation measurement) may then be determined based on the light measured by the light-sensitive element 114 when the target region of the individual 106 was not subjected to light produced by the first light source 108 (e.g., the predetermined amount of light).

In specific embodiments, determining the pulse oximetry measurement in step 350 can include: (i) determining a skin pigmentation value for the target region of the individual 106 based on the light measured by the spectroscopy device 110 when the target region was subjected to the predetermined amount of light produced by the first light source 108; (ii) selecting an R-curve based on the skin pigmentation value determined for the target region of the individual 106; and determining a pulse oximetry measurement (e.g., a blood oxygen saturation measurement) for the individual 106 based on the R-curve selected and the light measured by the light-sensitive element 114 when the target region was subjected to the at least two different wavelengths of light produced by the set of second light sources 112A, 112B.

As mentioned above, the calibrated pulse oximetry measurement taken using the pulse oximetry device 108 may be a blood oxygen saturation measurement ($SpO_2$), which can be determined based on the attenuation of light from the set of second light sources 112A, 112B. However, it should be appreciated that because of differences in light sources, sensors, and other factors, the pulse oximetry device 102 may need to be calibrated. In certain embodiments, an R-curve may be defined and used to determine an $SpO_2$ measurement based on the light measured from the set of second light sources 112A, 112B.

In embodiments, an R-curve may plot blood oxygen saturation against an R-value, which is determined as a function of the ratio of the AC and DC signals produced the set light sources 112A, 112B using the light-sensitive element 114. For example, in certain embodiments, the R-value may be determined according to Equation 1:

$$R = \frac{AC_{red}/DC_{red}}{AC_{IR}/DC_{IR}} \quad \text{(Equation 1)}$$

wherein $AC_{red}$ is the AC component of the signal produced by a red LED light source 112B, $DC_{red}$ is the DC component of the signal produced by the red LED light source 112B, $AC_{IR}$ is the AC component produced by an infrared LED light source 112A, and $DC_{IR}$ is the DC component of the signal produced by the infrared LED light source 112A.

It should be appreciated that each AC component of the signals produced by each light source of the set of second light sources 112A, 112B corresponds to the light absorbed by the blood flowing through a blood vessel at the target site, while each DC component of the signals produced by each light source of the set of second light sources 112A, 112B corresponds to light absorbed by other blood and tissues located at the target site.

In embodiments, the R-curve can be selected from an R-curve database storing a plurality of predefined R-curves, wherein each R-curve corresponds to one or a range of skin pigmentation values. For example, once a skin pigmentation value is determined for the target region of an individual 106 using the pulse oximetry device 102, the skin pigmentation value may looked-up in an R-curve database and used to select a predefined R-curve. In certain embodiments, each predefined R-curve stored in the R-curve database may correspond to a specific skin pigmentation value, or may cover a range of skin pigmentation values.

In further embodiments, instead of an R-curve, a set of equations or an algorithm may be developed based on different skin pigmentations and used to convert measurements from the light-sensitive element 114 into a blood oxygen saturation percentage ($SpO_2$ measurement). For example, instead of an R-curve database, the memory 204 may include a stored set of equations or algorithm that can receive as an input a skin pigmentation value / measurement and calibrate the conversion of measurements from the light-sensitive element 114 into an $SpO_2$ measurement. In such embodiments, instead of selecting a particular R-curve based on the skin pigmentation value, a particular conversion equation or a particular algorithm may be selected based on the skin pigmentation value and used to determine the $SpO_2$ measurement. In embodiments, each conversion equation or algorithm may correspond to one or a range of different skin pigmentation values.

According to the present disclosure, it should be understood that one or more steps of the methods 300 described herein may be performed using a calibration module 104. In particular embodiments, the calibration module 104 may operate the pulse oximetry device 102 to perform steps 310-340. The calibration module 104 may also receive electrical signals produced by the spectroscopy device 110 and the light-sensitive element 114 as described in steps 320, 340 when the corresponding light sources 108, 112A, 112B are operated. In further embodiments, the calibration module 104 can determine a pulse oximetry measurement (e.g., a blood oxygen saturation measurement) based on the electrical signals received from the spectroscopy device 110 and the light-sensitive element 114, as described in step 350.

Additionally, as shown in Fig. 3, the method 300 can include, in a step 360, displaying the pulse oximetry measurement taken using the pulse oximetry device 102. For example, as described above, the calibration module 104 can include a display 210 that can be used to display pulse oximetry measurements. In particular embodiments, the pulse oximetry measurement is a blood oxygen saturation measurement that is displayed on the display 210 at a percentage.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semiclosed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A system (100) for dynamically calibrating pulse oximetry measurements based on skin pigmentation, the system (100) comprising:

   a pulse oximetry device (102) comprising:

   a first light source (108) configured to subject a target region of an individual (106) to a predetermined amount of light;
   a spectroscopy device (110) configured to measure light produced by the first light source (108), wherein the light measured by the spectroscopy device (110) includes at least a portion of the predetermined amount of light that is either transmitted through and/or reflected by the target region of the individual (106);
   a set of second light sources (112A, 112B) configured to subject the target region of the individual (106) to at least two different wavelengths of light; and
   a light-sensitive element (114) configured to measure light produced by the set of second light sources (112A, 112B), wherein the light measured by the light-sensitive element (114) includes light that is either transmitted through and/or reflected by the target region of the individual (106); and
   a calibration module (104) in communication with the pulse oximetry device (102), wherein the calibration module (104) is configured to receive electrical signals generated by the spectroscopy device (110) and the light-sensitive element (114) based on the light measured respectively, and further configured to determine a blood oxygen saturation measurement based on the electrical signals received from the spectroscopy device (110) and the light-sensitive element (114).

2. The system (100) of claim 1, wherein the electrical signals generated by the spectroscopy device (110) and the light-sensitive element (114) are representative of an amount of light measured by the spectroscopy device (110) and the light-sensitive element (114), respectively.

3. The system (100) of claim 2, wherein the pulse oximetry device (102) is calibrated based on the light measured by the spectroscopy device (110) and the blood oxygen saturation measurement is determined based on the light measured by the light-sensitive element (114).

4. The system (100) of claim 3, wherein the blood oxygen saturation measurement is determined based on the light measured by the light-sensitive element (114) and an R-curve selected based on the light measured by the spectroscopy device (110).

5. The system (100) of claim 4, wherein the calibration module (104) comprises an R-curve database storing a plurality of R-curves, each R-curve corresponding to one or a range of skin pigmentations.

6. The system (100) of claim 5, wherein the calibration module (104) is configured to select an R-curve from the plurality of R-curves stored in the R-curve database based on the light measured by the spectroscopy device (110).

7. The system (100) of claim 1, wherein the calibration module (104) is configured to determine the blood oxygen saturation measurement based on the electrical signals received from the light-sensitive element (114) when the target region was not subjected to the predetermined amount of light produced by the first light source (108).

8. A computer program product (224) comprising: a non-transitory computer-readable storage medium (204) having stored thereon computer-readable instructions (222) that, when executed by one or more processors, cause the one or more processors to perform the following operations: (i) subjecting a target region of an individual to a predetermined amount of light using a first light source of a pulse oximetry device; (ii) measuring light produced by the first light source using a spectroscopy device, wherein the light measured by the spectroscopy device includes at least a portion of the predetermined amount of light that is either transmitted through and/or reflected by the target region of the individual; (iii) subjecting the target region of the individual to at least two different wavelengths of light using a set of second light sources of the pulse oximetry device; (iv) measuring light produced by the set of second light sources using a light-sensitive element, wherein the light measured by the light-sensitive element includes light that is either transmitted through and/or reflected by the target region of the individual; and (v) determining a blood oxygen saturation measurement for the individual based on the light measured by the spectroscopy device and the light measured by the light-sensitive element.

9. The computer program product (224) of claim 8, wherein determining the blood oxygen saturation measurement for the individual includes: (a) determining a skin pigmentation value for the target region of the individual based on the light measured by the spectroscopy device when the target region was subjected to the predetermined amount of light produced by the first light source; (b) selecting an R-curve based on the skin pigmentation value determined for the target region of the individual; and (c) determining the blood oxygen saturation measurement for the individual based on the R-curve selected and the light measured by the light-sensitive element when the target region was subjected to the at least two different wavelengths of light produced by the set of second light sources.

10. The computer program product (224) of claim 9, wherein the R-curve is selected from an R-curve database storing a plurality of predefined R-curves, each R-curve corresponding to one or a range of skin pigmentation values.

11. The computer program product (224) of claim 8, wherein the blood oxygen saturation measurement is determined based on the light measured by the light-sensitive element when the target region was not subjected to the predetermined amount of light produced by the first light source.

12. A method (300) for measuring a physiological parameter using a pulse oximetry device comprising a first light source, a spectroscopy device, a set of second light sources, and a light-sensitive element, the method comprising:

subjecting (310) a target region of an individual to a predetermined amount of light using the first light source of the pulse oximetry device; measuring (320) light produced by the first light source using the spectroscopy device of the pulse oximetry device, wherein the light measured by the spectroscopy device includes at least a portion of the predetermined amount of light that is either transmitted through and/or reflected by the target region of the individual; subjecting (330) the target region of the individual to at least two different wavelengths of light using the set of second light sources of the pulse oximetry device;

measuring (340) light produced by the set of second light sources using the light-sensitive element of the pulse oximetry device, wherein the light measured by the light-sensitive element includes light that is either transmitted through and/or reflected by the target region of the individual; and

determining (350) a blood oxygen saturation measurement for the individual based on the light measured by the spectroscopy device and the light measured by the light-sensitive element.

13. The method (300) of claim 12, wherein determining (350) the blood oxygen saturation measurement for the individual includes:

determining a skin pigmentation value for the target region of the individual based on the light measured by the spectroscopy device when the target region was subjected to the predetermined amount of light produced by the first light source;

selecting an R-curve based on the skin pigmentation value determined for the target region of the individual; and

determining the blood oxygen saturation measurement for the individual based on the R-curve selected and the light measured by the light-sensitive element when the target region was subjected to the at least two different wavelengths of light produced by the set of second light sources.

14. The method (300) of claim 13, wherein the R-curve is selected from an R-curve database storing a plurality of predefined R-curves, each R-curve corresponding to one or a range of skin pigmentation values.

15. The method (300) of claim 12, wherein the blood oxygen saturation measurement is determined based on the light measured by the light-sensitive element when the target region was not subjected to the predetermined amount of light produced by the first light source.

FIG. 1

EP 4 529 830 A1

FIG. 2

300

```
310 ──▶ ┌─────────────────────────────────┐
        │  SUBJECT TARGET TO LIGHT USING A FIRST │
        │         LIGHT SOURCE            │
        └─────────────────────────────────┘
                        │
                        ▼
320 ──▶ ┌─────────────────────────────────┐
        │  MEASURE LIGHT TRANSMITTED / REFLECTED │
        │   BY THE TARGET USING A SPECTROSCOPY │
        │             DEVICE              │
        └─────────────────────────────────┘
                        │
                        ▼
330 ──▶ ┌─────────────────────────────────┐
        │    SUBJECT TARGET TO AT LEAST TWO  │
        │  DIFFERENT WAVELENGTHS OF LIGHT USING A │
        │        SET OF LIGHT SOURCES     │
        └─────────────────────────────────┘
                        │
                        ▼
340 ──▶ ┌─────────────────────────────────┐
        │  MEASURE LIGHT TRANSMITTED / REFLECTED │
        │  BY THE TARGET USING A LIGHT-SENSITIVE │
        │            ELEMENT              │
        └─────────────────────────────────┘
                        │
                        ▼
350 ──▶ ┌─────────────────────────────────┐
        │  DETERMINE A BLOOD OXYGEN SATURATION │
        │   MEASUREMENT BASED ON THE LIGHT │
        │  MEASURED BY THE SPECTROSCOPY DEVICE │
        │   AND THE LIGHT-SENSITIVE ELEMENT │
        └─────────────────────────────────┘
                        │
                        ▼
360 ──▶ ┌─────────────────────────────────┐
        │   DISPLAY MEASUREMENT FROM PULSE │
        │        OXIMETRY DEVICE          │
        └─────────────────────────────────┘
```

# FIG. 3

Europäisches Patentamt

European Patent Office

Office européen des brevets

# EUROPEAN SEARCH REPORT

**Application Number**

EP 23 21 4169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/190115 A1 (RUSSO KATHRYN H [US]) 22 June 2023 (2023-06-22) * paragraphs [0005], [0035] - [0045]; claim 9; figures 1, 2A * | 1-15 | INV. A61B5/00 A61B5/145 A61B5/1495 |
| X | US 2023/103406 A1 (MEEHAN CHRISTOPHER J [US] ET AL) 6 April 2023 (2023-04-06) * paragraphs [0053] - [0055], [0061] - [0066] * | 1-15 | |

**TECHNICAL FIELDS SEARCHED (IPC)**

A61B

The present search report has been drawn up for all claims

| Place of search | Date of completion of the search | Examiner |
|---|---|---|
| The Hague | 30 April 2024 | Furlan, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 21 4169**

This annex lists the patent family members relating to the patent documents cited in the above-mentioned European search report.
The members are as contained in the European Patent Office EDP file on
The European Patent Office is in no way liable for these particulars which are merely given for the purpose of information.

**30-04-2024**

| Patent document cited in search report | Publication date | Patent family member(s) | Publication date |
|---|---|---|---|
| US 2023190115 A1 | 22-06-2023 | NONE | |
| US 2023103406 A1 | 06-04-2023 | NONE | |

For more details about this annex : see Official Journal of the European Patent Office, No. 12/82